# EUROPEAN PATENT APPLICATION

(11) **EP 1 837 055 A1**
(43) Date of publication of application: **26.09.2007**
(21) Application number: 07075110.2
(22) Date of filing: 06.02.2007
(51) Int. Cl.: A61Q 19/00, A61K 8/36, A61K 8/67, A61K 8/34

(54) **Skin care cream**

(30) Priority: 06.02.2006 NL 1031084
(71) Applicant: Hoogenboom, Bob, 1171 LD Badhoevedorp (NL)
(72) Inventor: Hoogenboom, Bob, 1171 LD Badhoevedorp (NL)
(74) Representative: Wittop Koning, Tom Hugo

(57) **Abstract**

The present invention relates to a cosmetic composition which can be administered topically. The composition is characterized in that the composition comprises at least 3 per cent by weight of alpha-lipoic acid and also vitamin E and at least one fatty alcohol. The invention furthermore relates to use of the compositions according to the invention for the treatment of disorders of the skin.

## Description

The present invention relates to cosmetic compositions for topical administration, a kit which comprises such a composition, use of such compositions, and a method for the production of such compositions.

The skin is the biggest organ of the human being, and just like internal organs it comprises different specialized cells which perform functions in the skin.

The skin is built up of different layers which consist of several tissues. The outermost layer is the epidermis and consists mainly of an epithelium. The dermis lies beneath the epidermis and comprises mainly collagen connective tissue.

One of the most important functions of the skin is to serve as a protective layer for the internal parts of the body. Under normal conditions this protective layer formed by the skin offers protection against harmful influences from outside, such as ultraviolet light, smoke, exhaust gases, parasites, and pathogens such as (microbial) infections, fungi, irritant substances and substances which cause allergies, but also against dehydration.

A healthy skin is thus of essential importance for the protection of the body. Consumers therefore regard a healthy skin as a beautiful skin, and keeping the skin healthy and beautiful is a daily occupation for many people.

During life the skin is subject to many changes and threats. Apart from influences from outside, such as sunlight for example, physical changes such as ageing or abnormalities elsewhere in the body can also lead to changes in the skin.

During the ageing process of the body or the skin, for example, the skin usually exhibits various visible changes. The skin becomes more flaccid, for example, starts to show wrinkles and lines, and irregularities, scars and colour changes may develop. Such changes in the skin can also occur if the skin is excessively exposed to sunlight.

Another threat to the skin is damage to the skin, as a result of which sores or scars can develop, for example.

A dry or rough skin can for example develop if the skin is no longer able to retain the right amount of moisture. Excessive washing with soap can also contribute to this problem.

A more serious disorder is for example psoriasis. This is a chronic skin disease which is characterized by thick red desquamating patches on the skin as a result of disturbances of skin growth. Whereas under normal conditions old skin cells are gradually replaced by new skin cells from the underside of the epidermis, in psoriasis the production of skin cells is accelerated and the cells created in excess are not rejected. This gives rise to the characteristic thick red desquamating patches on the skin.

Acne is a collective name for various skin disorders which manifest themselves in the form of pustules on the skin. The best-known form is the so-called "adolescence spots", which may be present on the head and neck, but also on other parts of the body, especially during puberty. This form of acne is thought to be due to excessive formation of sebum. Scars and small sores can develop on the skin as a result of acne.

Other commonly occurring examples of changes in the skin are the development of pigment marks, such as liver spots (yellowish brown to black spots on the skin), lentigo senilis and the like.

One of the most widely used ways of preventing or combating such changes is the use of so-called skin care creams. Such products must usually be applied to the skin, usually daily and for prolonged periods, for example to that part of the skin where an unwanted change has taken place or may take place. Such products for cosmetic treatment of the skin in order to prevent or control the above skin problems are known in the prior art. Many sorts of creams, oils, ointments, lotions, masks and the like are available on the market for topical application. From time immemorial such products have usually comprised adipoid or cutaneous substances such as lipids (from olive oil for example) and moisture, usually in the form of emulsions, and have usually had the aim of making good deficiencies in the skin while at the same time applying a temporary protective layer to the skin.

More recently products to which "active components" have been added, such as vitamins and antioxidants, or herb extracts, have been brought onto the market. It is claimed that such components help to keep the skin healthy or accelerate the restoration of the skin. Such components thus do not usually serve to make good existing deficiencies in the skin, but affect the processes which take place in the skin.

US 2005/0118283 for example describes the use of various oxidants such as *inter alia* carotenoid extract of green tea, extract of Rooibos and grapestone extract in compositions for skin care, in order to control or prevent free radicals which occur in the skin and contribute to the development of the above-mentioned skin disorders and changes, for example. Such compositions are preferably taken orally, although topical application is also possible according to the inventors.

US 2003/0091605 describes the presence of retonoids (Vitamin A acid) or vitamin A in products for the care of the ageing skin.

Another known component which is used in skin care agents that are applied topically is lipoic acid. US 2002/0012642 describes a method for preventing skin damage by making use of lipoic acid in a dermatologically acceptable carrier that can be applied topically to the skin. Lipoic acid can be combined with vitamin E (and derivatives thereof), with the application stating that preferably amounts from 0.1 wt% to approximately 5 wt% can be used.

WO 97/10808 describes a composition which comprises 0.25-5 wt% alpha-lipoic acid. The composition preferably comprises 1-3 wt% alpha-lipoic acid. Tocotrienol can be added to the composition comprising alpha-lipoic acid. According to the inventors the combination of both compounds is advantageous because alpha-lipoic acid is said to strengthen the action of tocotrienol in the skin.

US 2003/0091605 describes the use of alpha-lipoic acid in the preparation of cosmetic or dermatological compositions for the restoration of the taut skin, in particular the ageing skin. Compositions which comprise amounts of alpha-lipoic acid up to a maximum of 2 wt% are described in the 25 different examples. Alpha-lipoic acid can be combined according to the invention with a large number of different compounds, such as antioxidants, UVB filters, medicaments, formulation agents such as viscosity-changing agents, colourants, surface-active compounds and the like, with no particular preference being given to any of these compounds.

As is apparent from the above, alpha-lipoic acid, alone or in combination with a wide variety of other compounds, is used in products for the treatment of the skin. A disadvantage of the products available today which are discussed above, however, is the fact that the amount of alpha-lipoic acid which is used in such products is relatively low (up to 2 wt%). It has been found that higher concentrations can lead to skin irritation and/or a stinging feeling on the skin and/or redness of the skin (hereinafter together called "skin irritation"). Because of the relatively low concentration in the products available the optimum effect of alpha-lipoic acid is not achieved. A disadvantage of this is that either treatments of longer duration or more treatments are needed to bring the skin to the desired condition, or that the desired result is not achieved. Moreover there are problems in product development with regard to the stabilization of higher concentrations of the active components, such as vitamin E and alpha-lipoic acid, against oxidative breakdown and/or separation, for example. As a result of this the life of the product is limited and such products are not suitable for use as skin care creams.

There is thus a need for a solution to the above problems, and to provide skin care creams in which alpha-lipoic acid is present in a higher amount than is now available, so that cosmetic treatment can be carried out more effectively.

The present inventor has carried out research and has surprisingly and unexpectedly found that a solution to at least one and/or several of the above problems can be provided with a cosmetic composition for topical administration, characterized in that the composition comprises at least 3 per cent by weight (wt%) of alpha-lipoic acid, vitamin E and at least one fatty alcohol.

Such a composition provides a high concentration of alpha-lipoic acid in a stable (for example no separation) skin care cream, without the stated problems with regard to skin irritation occurring.

The composition according to the invention comprises at least 3 wt% of alpha-lipoic acid, relative to the total weight of the composition. The term "alpha-lipoic acid" is known to the person skilled in the art, and this compound is also described as ALA, thioctic acid, lipoic acid, 1,2-dithiacylcyclopentan-3-valeric acid, and/or 1,2-dithiolane-3-pentanoic acid. The person skilled in the art knows that the term alpha-lipoic acid must also be understood to include salts and derivatives thereof.

The composition according to the invention furthermore comprises vitamin E, in particular tocopheryl acetate and/or tocotrienol. The person skilled in the art knows that the term vitamin E includes all tocopherols and tocotrienols and derivatives thereof which exhibit the biological activity of d-alpha-tocopherol, as well as any combination thereof. Examples of this are for example tocopheryl acetate, tocopherols and also tocomonoenol. It has surprisingly been found that by combining high concentrations of alpha-lipoic acid (for example more than 3 wt%) with a suitable amount of vitamin E in the composition according to the invention, the skin irritation and/or stinging feeling on the skin and/or redness of the skin which can occur when a high concentration of alpha-lipoic acid is applied does not occur, or at any event occurs to a reduced extent.

With regard to the above, reference is made to DE 10111048. In two examples (10 and 16) this document describes a combination of alpha-lipoic acid and tocopherol. The wt% of alpha-lipoic acid in these compositions, however, is much less than 3 wt%, and the problem of a higher concentration of alpha-lipoic acid, as described above, will not occur at these lower concentrations and is also not described as such. In the other 13 examples which are described in this document, too, at most 2 wt% of alpha-lipoic acid in a composition is used in combination with a multitude of other usual components.

DE 10111045 and DE 10111047 also describe the combination of alpha-lipoic acid and tocopherol in various examples, but in keeping with DE 10111048 these documents, too, only describe combinations in which the wt% of alpha-lipoic acid is far below the level of 3 wt%. As has already been explained above, no irritation of the skin as a result of the alpha-lipoic acid will occur at these lower concentrations. Thus these documents too do not describe the problem that can occur as a result of higher concentrations of components, or the solution thereto according to the present invention.

Reference is furthermore made to WO 03/086329. This application describes a composition for preventing skin damage induced by free radicals. The compositions comprise ascorbic acid in combination with tocopherol and at least one antioxidant chosen from a group consisting of nine different compounds, including alpha-lipoic acid (Claim 1 of WO 03/086329). In Example 8 of the PCT application a composition is described which comprises vitamin E (1 wt%) and alpha-lipoic acid (0.5 wt%), but in this application, too, the amount of alpha-lipoic acid is far below the level at which irritation of the skin can be expected. This application, too, does not describe the problem of the higher concentrations of alpha-lipoic acid, nor does it offer any solution thereto.

Reference is made last of all to WO 2004/060338. This application relates to a composition for the treatment of the skin and to gloves which are provided with such a composition. Table 1 shows components which can be used in the composition and the amounts in wt%. Thus 0.5-10 wt% of vitamin A, vitamin B, vitamin E, alpha-lipoic acid, eucalyptus and jojoba can be used, for example. Here too, however, the problem of the occurrence of irritation on the skin as a result of higher concentrations of alpha-lipoic acid is not described. Nor does the document give any solution to such a problem. The document does not describe any single specific example with for example at least 3 wt% of alpha-lipoic acid in combination with an effective amount of vitamin E. In other words, compositions according to the present invention are not specifically described, nor does the document give any cause to arrive at such a composition.

The composition according to the invention furthermore comprises at least one fatty alcohol. The term "fatty alcohol" is known to the person skilled in the art, and relates to primary alcohols of linear carbon chains, such as fatty acids for example. Such a fatty alcohol can be ethoxylated or propoxylated and the like. It has been found that addition of a suitable amount of at least one fatty alcohol to the combination of a high concentration of alpha-lipoic acid and a suitable amount of vitamin E advantageously leads to the formation of a stable composition, without the alpha-lipoic acid and the vitamin E separating appreciably or becoming inactive (through oxidation for example), and moreover the effect of the non-occurrence, or at any event reduced occurrence, of skin irritation and/or a stinging feeling on the skin and/or redness of the skin is maintained. With the composition according to the invention it is now advantageously possible for the first time to provide skin care creams which comprise high concentrations of alpha-lipoic acid, and do not exhibit the disadvantages referred to above, as a result of which the skin can be cared for and treated more effectively and efficiently with the composition according to the invention.

In a preferred embodiment the composition according to the invention is characterized in that the fatty alcohol is chosen from the group consisting of cetyl alcohol, palmitoyl alcohol, (iso)stearyl alcohol and/or cetearyl alcohol and combinations thereof, the fatty alcohol is preferably cetearyl alcohol. Such fatty alcohols are known to the person skilled in the art. Cetearyl alcohol is for example also known under the name cetyl stearyl alcohol or ceto-stearyl alcohol and is a mixture comprising fatty alcohols with carbon chains of 16 and 18 carbon atoms (C16 and C18). The ratio between C16 and C18 can for example lie between 3:1 and 1:4, and is usually approximately 1:3. It has been found that the above fatty alcohols can be used with advantage in the composition according to the invention and that the combination of a high concentration of alpha-lipoic acid and vitamin E is especially efficient and effective if such fatty alcohols, preferably cetearyl alcohol, are used in the composition.

In a further embodiment the composition according to the invention is characterized in that the composition, in addition to at least 3 wt% of alpha-lipoic acid and vitamin E, comprises more than 5 wt% (relative to the total weight of the composition) of fatty alcohol, preferably more than 10 wt% of fatty alcohol. It has been found that at a wt% for the fatty alcohol higher than 5 wt%, preferably higher than 10 wt%, the composition according to the invention is especially effective and can be used with advantage as a skin care cream.
In yet another preferred embodiment the composition according to the invention comprises more than 5 wt% of alpha-lipoic acid, preferably more than 6 wt%, most preferably more than 7.5 wt% of alpha-lipoic acid. Alpha-lipoic acid is a powerful antioxidant and is soluble in both water and fat. A special property of alpha-lipoic acid is that it does not act only as an antioxidant, notably protecting other antioxidants such as carotenoids, vitamin C and E, which are for example present in the skin, and being able to combat the formation of free radicals, but also being capable of reducing antioxidants that have already been oxidized. Alpha-lipoic acid can therefore return ('recycle') other antioxidants to their original state and as a result appreciably increase the effectiveness of antioxidants. It is thought that alpha-lipoic acid is as a result effective in the care of the skin and in the treatment and/or prevention of skin disorders. It has been found that the composition according to the invention advantageously comprises more than 5 wt%, preferably more than 6 wt%, most preferably more than 7.5 wt% of alpha-lipoic acid. As a result of the high concentrations in the composition according to the invention effective care of the skin can take place without the problems of the prior art (red skin, irritation and stinging feeling) occurring.

According to a further embodiment the composition according to the invention comprises more than 1 wt% (relative to the total weight of the composition), preferably more than 2 wt%, more preferably more than 3 wt% of vitamin E. It has been found that at vitamin E concentrations lower than 1 wt% there is reduced effectiveness of the composition according to the invention, and for example the stinging feeling or the skin irritation from high concentrations of alpha-lipoic acid in the composition according to the invention is prevented less well. Vitamin E in the concentrations stated above, in combination with a high concentration of alpha-lipoic acid and at least one fatty alcohol, as described above, exhibits especial effectiveness in the care of the skin and in the prevention and/or treatment of skin disorders.

It has been found that the ratio between the various components of the composition according to the invention is important for optimal efficacy. According to a further preferred embodiment the composition according to the invention is characterized in that the wt% ratio between alpha-lipoic acid and vitamin E lies in the range from 0.1 to 5.0, preferably 0.2 to 4.4, more preferably between 0.4 and 2.2. If the ratio of both compounds deviates to a significant extent from the ratios found, the effectiveness of the composition will decrease (at a wt% ratio of 10 or more for example).

It has accordingly been found that the ratio between alpha-lipoic acid and the at least one fatty alcohol in the composition according to the invention is of importance for optimum efficacy of the composition. In one embodiment of the composition according to the invention the wt% ratio between alpha-lipoic acid and the at least one fatty alcohol lies in the range 0.1-1.4, preferably in the range 0.15-0.9, more preferably in the range 0.2-0.8.

In an especially preferred embodiment the composition according to the invention comprises at least 7-9 wt% of alpha-lipoic acid, 3-5 wt% of vitamin E and 10-14 wt% of ceterayl alcohol. Such compositions exhibit exceptionally good properties in skin care and/or in the control or prevention of skin disorders.

The person skilled in the art will understand that in addition to the components according to the invention the composition can comprise further customary components of skin care creams. Customary components of skin care creams are known to the person skilled in the art and can without further inventive thought be present in suitable amounts in the composition according to the invention. Suitable customary components can *inter alia* be chosen from for example other antioxidants, fats, extracts of plants, such as for example Aloe Vera, DMAE (dimethylaminoethanol), ceramides, phospholipids, fatty acids, DHLA, pH-regulating components, micronutrients, vitamins, alpha-hydroxy acids such as citric acid, beta-hydroxy acids, such as salicylic acid, hydroquinones and other depigmenting components, kojic acid, retinol, vitamin C, hyaluronic acid, hydrating components, liposomes and the like. In a most preferred embodiment the composition according to the invention comprises:

| Component | Synonym | Gr/kg of product |
|---|---|---|
| Cetiol V | decyloleate | 144.24 |
| Lanette SX | Cetearyl alcohol and sodium cetearyl sulphate | 115.88 |
| Silicon oil | dimethicone | 10.00 |
| Demiwater | aqua | 462.46 |
| glycerolum | Glycerine | 30.9 |
| DMAE | | 40.0 |
| ascorbyl palmitate | | 40.0 |
| Coenzyme 10 | ubiquitone | 16.0 |
| Alpha-lipoic acid | | 80.0 |
| Tocopheryl acetate | | 40.0 |
| 1-tyrosine | | 12.8 |
| preservatives | Phenoxyethanol, methylparaben, ethylparaben, propylparaben and/or isobutylparaben | 8.0 |

In another aspect the invention relates to a kit for topical administration on the skin, characterized in that the kit comprises:
a) a first composition according to the invention, characterized in that the composition comprises 4-10 wt% of alpha-lipoic acid and 2-8 wt% of vitamin E; and
b) a second composition according to the invention, characterized in that the composition comprises at least 3-8 wt% of alpha-lipoic acid and 4-10 wt% of vitamin E.

Such a kit has been found to be in particular suitable for use as a multi-phase product. The first composition is for example employed at the start of skin care, while the second composition can be employed subsequently. Use of the first composition during 7 days or weeks, for example, provides higher concentrations of alpha-lipoic acid and other components and can in particular be suitably used in order to accelerate or bring about restoration of the skin at the start of the care of the skin and/or the treatment of skin disorders, after which the second composition can in particular be suitably employed in order to maintain or further improve the equilibrium in the skin. Such a kit is in particular suitable as a so-called initiation and maintenance kit. The person skilled in the art knows that the duration of the use of the first composition is dependent on the nature of the disorder and the desired result. The person skilled in the art knows that the composition of the first composition may be different from the composition of the second composition. The first composition may for example comprise further components which are useful in the first phase of skin care, for example because such components accelerate or improve restoration, whereas such components in a second composition are not useful or less useful, and vice versa.

According to another aspect the invention relates to the use of a composition according to the invention for the cosmetic treatment of the skin. The term "cosmetic treatment" means treatment for the purpose of increasing or maintaining the beauty of the body, without there having to be any medical need or indication for it. Examples of cosmetic treatment are for example the treatment of wrinkles and scars, and the treatment of mild forms of acne. A special example of cosmetic treatment concerns the prevention of changes to the skin, such as the prevention or limitation of skin ageing or the prevention or limitation of the development of liver spots or lentigo senilis.

According to another aspect the invention relates to use of a composition according to the invention for the production of a medicament for the treatment of the skin.

The composition is preferably used for a disorder of the skin chosen from the group consisting of acne, psoriasis, scars, liver spots, skin ageing and/or skin damage. It has been found that the composition according to the invention is in particular effective for reducing the symptoms and for restoring the skin in said disorders of the skin. As has been indicated above, the composition according to the invention is suitable for use in both cosmetic and medical treatment.

According to another aspect the invention relates to a method for production of a composition according to the invention, characterized in that the method comprises the steps of mixing alpha-lipoic acid and vitamin E with the other components of the composition at a temperature below 30 °C, preferably below 25 °C, more preferably below 20 °C, and most preferably below room temperature. It has been found that in the production of a composition according to the invention which comprises high concentrations of alpha-lipoic acid and vitamin E, mixing at reduced temperature does not negatively affect the efficacy of the composition, or does so only to a limited degree. At higher temperatures, and those which can customarily be employed in the production of skin care creams (40 °C for example), it is found that the efficacy of the composition is negatively affected. According to another embodiment of the method, prior to the addition of alpha-lipoic acid and vitamin E, the remaining components are mixed at a temperature above 20 °C, preferably above 25 °C, more preferably above 30 °C, and most preferably above room temperature, after which after cooling alpha-lipoic acid and vitamin E are added and mixed at reduced temperature. The person skilled in the art will understand that components which lose activity during mixing at a temperature above 30 °C are preferably mixed together with alpha-lipoic acid and vitamin E at a temperature below 30 °C, preferably below 25 °C, more preferably below 20 °C, and most preferably below room temperature.

The present invention thus provides for the use of at least vitamin E and at least one fatty alcohol as a combination for the prevention of problems with high concentrations of alpha-lipoic acid in a cosmetic composition. The use of vitamin E, especially in the amounts, concentrations and wt% described herein, and fatty alcohol, especially in the amounts, concentrations and wt% described herein, in a cosmetic composition for topical administration which comprises at least 3 wt% of alpha-lipoic acid (thus in amounts which are described herein for alpha-lipoic acid and which comprises at least 3 wt% in the composition) is in particular provided for. Application of a combination of at least vitamin E and at least one fatty alcohol in a cosmetic composition for topical administration which comprises at least 3 wt% of alpha-lipoic acid is provided for.

In another aspect according to the invention provision is made for a method for the prevention of the problems described above with higher concentrations of alpha-lipoic acid (at least 3 wt% for example) which consists of the step of applying a cosmetic composition for topical administration which comprises at least 3 wt% of alpha-lipoic acid and comprises at least vitamin E and at least one fatty alcohol. The person skilled in the art will understand that the amounts of vitamin E and fatty alcohol which are used in such a method can be the amounts, concentrations and ratios such as have been described herein.

The invention will be further explained below on the basis of examples, without any limitation being imposed in so doing.

### Example 1

A skin care cream with the composition given in Table 1 was produced.

**Table 1:**

| Component | Synonym | Gr/kg of product |
|---|---|---|
| Cetiol V | decyloleate | 144.24 |
| Lanette SX | Cetearyl alcohol and sodium cetearyl sulphate | 115.88 |
| Silicon oil | dimethicone | 10.00 |
| Demiwater | aqua | 462.46 |
| glycerolum | Glycerine | 30.9 |
| DMAE | | 40.0 |
| ascorbyl palmitate | | 40.0 |
| Coenzyme 10 | ubiquitone | 16.0 |
| Alpha-lipoic acid | | 80.0 |
| Tocopheryl acetate | | 40.0 |
| 1-tyrosine | | 12.8 |
| preservatives | Phenoxyethanol, methylparaben, ethylparaben, propylparaben and/or isobutylparaben | 8.0 |

### Example 2

### Composition kit

The kit consists of a first and a second composition. The composition was produced from a base composition as set out below:

| Component | Synonym | Gr/kg of product |
|---|---|---|
| Cetiol V | decyloleate | 187 |
| Lanette SX | Cetearyl alcohol and sodium cetearyl sulphate | 150 |
| Silicon oil | dimethicone | 13 |
| Demiwater | aqua | 600 |
| glycerolum | Glycerine | 40 |
| preservatives | Phenoxyethanol, methylparaben, ethylparaben, propylparaben and/or isobutylparaben | 10.0 |

The first composition comprises:

| Component | Synonym | Gr/kg of product |
|---|---|---|
| base composition | | 771.2 |
| DMAE | | 40.0 |
| ascorbyl palmitate | | 40.0 |
| Coenzyme 10 | ubiquitone | 16.0 |
| Alpha-lipoic acid | | 80.0 |
| Tocopheryl acetate | | 40.0 |
| 1-tyrosine | | 12.8 |

The second composition comprises:

| Component | Synonym | Gr/kg of product |
|---|---|---|
| base composition | | 798.4 |
| DMAE | | 30.0 |
| ascorbyl palmitate | | 30.0 |
| Coenzyme 10 | ubiquitone | 12.0 |
| Alpha-lipoic acid | | 60.0 |
| Tocopheryl acetate | | 60.0 |
| 1-tyrosine | | 9.6 |

The kit according to the invention is especially suitable for use in the treatment of skin disorders.

### Example 3

A composition according to the invention was produced from the base composition according to Example 2.
The composition comprises:

| Component | Synonym | Gr/kg of product |
|---|---|---|
| base composition (see Example 2) | | 825.6 |
| DMAE | | 20.0 |
| ascorbyl palmitate | | 20.0 |
| Coenzyme 10 | ubiquitone | 8.0 |
| Alpha-lipoic acid | | 40.0 |
| Tocopheryl acetate | | 80.0 |
| 1-tyrosine | | 6.4 |

The composition is especially suitable for use in the treatment, for example cosmetic treatment, of the skin, in particular the sensitive skin.

## Claims

1. Cosmetic composition for topical administration, **characterized in that** the composition comprises at least 3 wt% of alpha-lipoic acid, vitamin E and at least one fatty alcohol.

2. Composition according to claim 1, **characterized in that** the fatty alcohol is chosen from the group consisting of cetyl alcohol, palmitoyl alcohol, (iso)stearyl alcohol and/or cetearyl alcohol and combinations thereof, preferably cetearyl alcohol.

3. Composition according to claim 1 or 2, **characterized in that** the composition comprises more than 5 wt% of fatty alcohol, preferably more than 10 wt% of fatty alcohol.

4. Composition according to any of claims 1-3, **characterized in that** the composition comprises more than 5 wt% of alpha-lipoic acid, preferably more than 6 wt%, most preferably more than 7.5 wt% of alpha-lipoic acid.

5. Composition according to any of the preceding claims, **characterized in that** the composition comprises more than 1 wt%, preferably more than 2 wt%, more preferably more than 3 wt% of vitamin E.

6. Composition according to any of the preceding claims, **characterized in that** the wt% ratio between alpha-lipoic acid and vitamin E lies in the range from 0.1 to 5.0, preferably 0.2 to 4.4, more preferably between 0.4 and 2.2.

7. Composition according to any of the preceding claims, **characterized in that** the wt% ratio between alpha-lipoic acid and the at least one fatty alcohol lies in the range from 0.1 to 1.4, preferably 0.15 to 0.9, more preferably 0.2-0.8.

8. Composition according to any of the preceding claims, **characterized in that** the composition comprises at least 7-9 wt% of alpha-lipoic acid, 3-5 wt% of vitamin E and 10-14 wt% of cetearyl alcohol.

9. Composition according to any of the preceding claims, **characterized in that** per kilogram of the composition the composition comprises:
- 145 gr of Cetiol V (decyloleate);
- 116 gr of Lanette SX (Cetearyl alcohol and sodium cetearyl sulphate);
- 10 gr of silicon oil (dimethicone);
- 462 gr of Demiwater (aqua);
- 31 gr of glycerolum (Glycerine);
- 40 gr of DMAE;
- 40 gr of ascorbyl palmitate;
- 16 gr of Coenzyme 10 (ubiquitone);
- 80 gr of alpha-lipoic acid;
- 40 gr of tocopheryl acetate;
- 12 gr of 1-tyrosine;
- 8 gr of preservatives (Phenoxy ethanol, methylparaben, ethylparaben, propylparaben and/or isobutylparaben).

10. Kit for topical administration on the skin, **characterized in that** the kit comprises:
a) a first composition according to any of the preceding claims, **characterized in that** the composition comprises 4-10 wt% of alpha-lipoic acid and 2-8 wt% of vitamin E; and
b) a second composition according to any of the preceding claims, **characterized in that** the composition comprises at least 3-8 wt% of alpha-lipoic acid and 4-10 wt% of vitamin E.

11. Use of a composition according to any of the preceding claims for the cosmetic treatment of the skin.

12. Use of a composition according to any of claims 1-10 for the production of a medicament for the treatment of the skin.

13. Use according to claim 11 or 12, **characterized in that** the skin has a disorder chosen from the group consisting of acne, psoriasis, scars, liver spots, skin ageing and/or skin damage.

14. Use according to claim 11-13 for the prevention of skin disorders.

15. Method for the production of a composition according to any of claims 1-10, **characterized in that** the method comprises the steps of mixing alpha-lipoic acid and vitamin E with the other components of the composition at a temperature below 30 °C.

16. Method according to claim 15, **characterized in that** the mixing is preceded by the mixing of the stated other components of the composition at a temperature above 30 °C.

17. Use of a combination of at least vitamin E and at least one fatty alcohol in a cosmetic composition for topical administration which comprises at least 3 wt% of alpha-lipoic acid.
